# EUROPEAN PATENT APPLICATION

(11) **EP 2 954 877 A1**
(43) Date of publication of application: **16.12.2015**
(21) Application number: 15166772.2
(22) Date of filing: 30.11.2011
(51) Int. Cl.: A61F 2/30, A61L 27/00

(54) **AN INTERFACE WALL SUITABLE FOR INTERFACING WITH AT LEAST ONE BONE TISSUE**

(30) Priority: 30.11.2010 IT PD20100360
(62) Divisional of application: 11807753.6
(71) Applicant: Guerra, Paolo, 20145 Milano (IT); Giuliani, Alessio, 20136 Milano (IT)
(72) Inventor: Guerra, Paolo, 20145 Milano (IT); Giuliani, Alessio, 20136 Milano (IT)
(74) Representative: Fiorentino, Luca

(57) **Abstract**

An interface wall (12) suitable for interfacing with at least one bone tissue, comprising a substrate (16) is provided. Advantageously, a first covering (20) comprising first functional groups (28) suitable to form chemical bonds, is applied to said substrate (16). On the side of the first covering (20) the substrate comprise protuberances (21,23,24,26) and/or recesses (22,25) suitable to encourage the attachment of said first functional groups (28). A second covering (32) comprising nanostructures is applied to the first covering (20), on the side opposite the substrate (16), and an interface covering (40) comprising molecules (44) is applied to the second covering (32) on the side opposite the first covering (20). Second functional groups (48) suitable to form chemical bonds, so as to strengthen the bonds between the second covering (32) and the interface covering (40) are inserted between the interface covering (40) and the second covering (32). Methods of producing such interface wall (12) are also provided.

## Description

The present invention relates to a prosthesis, in particular a multifunctional prosthesis with multilayer covering, having an improved and differentiated biological response, and method of producing thereof.

In particular it is known of in the prosthetics sector to implant prostheses, e.g. orthopaedic, trying to favour the osseointegration or the osseous encapsulation of the prosthesis as far as possible.

Osseointegration is essential for restoring the biomechanical functions of the prosthesized bone as soon as possible.

Not infrequently the need arises to service the implanted prosthesis: in practice this involves explanting the prosthesis to replace, reposition or modify it and adapt it better to the specific anatomical conformation of the patient.

The explant phase of the prosthesis is highly invasive in that the prosthesis, thanks to its osseointegration is firmly bound both to the cortex and to the inner spongy part of the bone.

As a result, the removal of prosthesis performed according to the prior art often proves excessively invasive for the patient.

It follows that in the art servicing operations are reduced to a minimum except where strictly necessary.

There is therefore a tendency to leave the prosthesis first implanted in the patient in position which does not always ensure optimal functioning for the patient.

In addition, one of the main causes of failure of the prosthetic implant is due to infections.

Such infections may occur subsequent to the implant and require removal of the prosthesis.

The need is therefore also felt to provide prostheses which limit the infections that are one of the main causes of failure of prosthesis implants.

The purpose of the present invention is to make a prosthesis which overcomes the drawbacks mentioned with reference to the prior art.

Such drawbacks and limitations are resolved by a prosthesis according to claim 1 and by a method of making a prosthesis according to claim 16.

Other embodiments of the prosthesis according to the invention are described in the subsequent claims.

Further characteristics and advantages of the present invention will be clearly comprehensible from the description given below of its preferred embodiments, made by way of a non-limiting example, wherein:

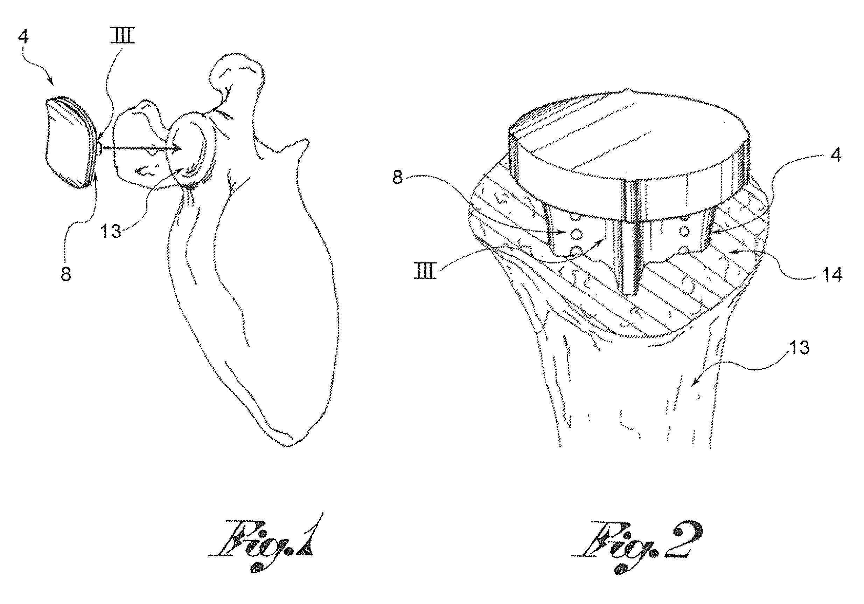

figures 1 and 2 shows perspective views of applications of prostheses according to possible embodiments of the present invention;
figure 3 shows an enlarged schematic view of the details III of the prosthesis in figures 1 and 2, according to one possible embodiment of the present invention;
figures 4-6 shows enlarged schematic views of the detail III in figures 1 and 2, according to embodiment variations of the present invention;
figure 7 shows an enlarged view of the detail VII in figure 6.

The elements or parts of elements common to the embodiments described below will be indicated using the same reference numerals.

With reference to the aforesaid figures, reference numeral 4 globally denotes a multifunctional prosthesis with multilayer covering.

The definition of prosthesis should be considered in the broad, not limited, sense; in other words any prosthesis suitable for interfacing with or replacing at least partially a bone or portion of bone is meant.

Merely by way of example, one might mean a glenoid, shoulder, knee or hip prosthesis, a prosthesis of a vertebral body and the like.

The prosthesis 4 comprises a prosthesis body 8 having at least one interface wall 12 suitable for interfacing with at least one bone tissue,

The term "interface" is taken to mean that the interface wall 12 of the prosthesis body 8, or a portion of it, is suitable for positioning itself in contact with a bone, e.g. with a cortex 13 of the bone, that is the external, rigid portion covering the bone and/or with the spongy part of the bone 14, that is the inner bone tissue covered by said cortex 13.

The interface wall 12 comprises a substrate 16.

According to one embodiment said substrate 16 is made from titanium or titanium alloy, such as a Ti6Al4V alloy, known for its biocompatibility.

The interface wall 12 need not necessarily be solid and in single material but may comprise a core in different material subsequently covered, e.g. titanium or titanium alloy at such substrate 16.

On the side of an associable first covering 20 and on the side opposite the interface wall 12, the substrate 16 comprises protuberances 21 and/or recesses 22.

Such protuberances 21 and/or recesses 22 may, in one embodiment, have a circular geometry e.g. cylindrical or spherical, suitable for encouraging attachment with first functional groups 28, as described further below.

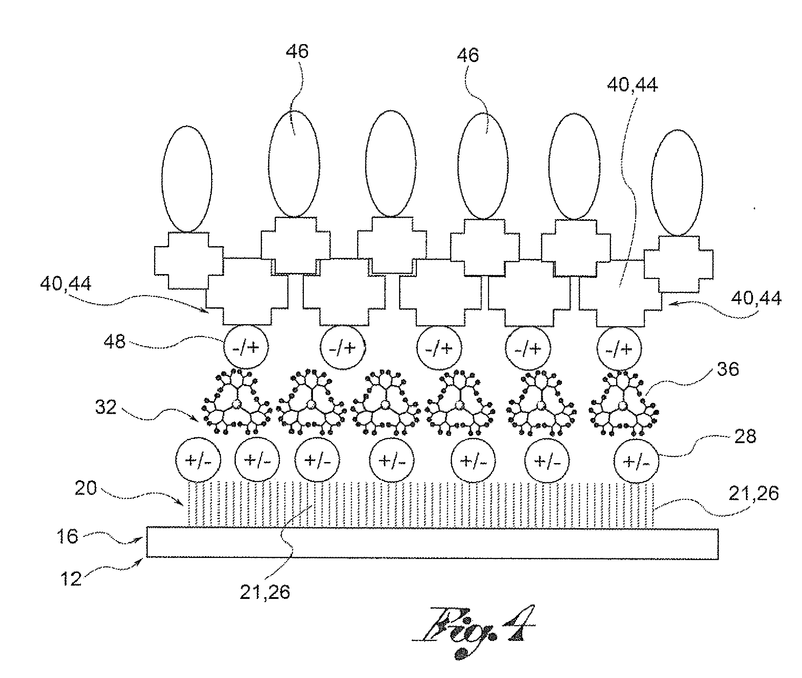

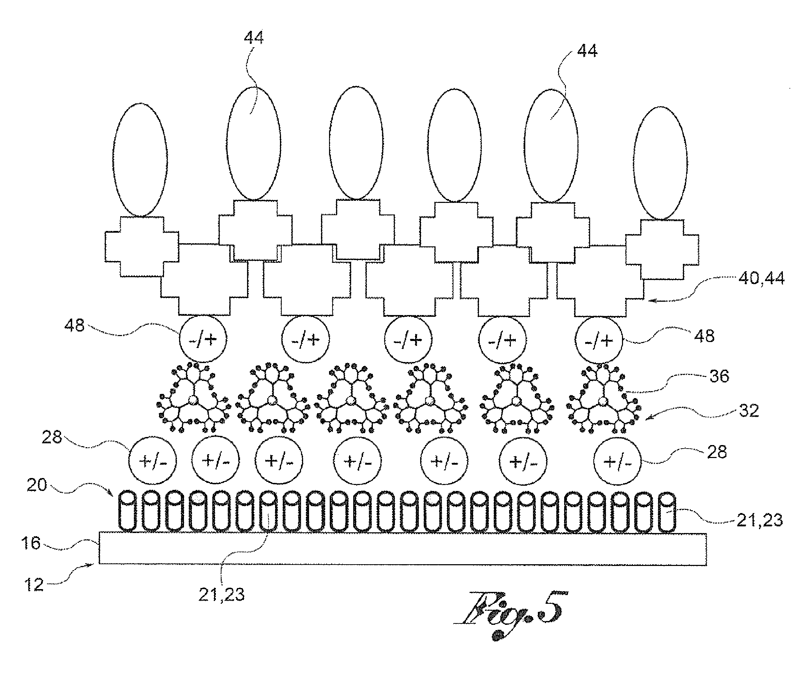

According to one embodiment said protuberances comprise nanotubes 23 or nanowires 26. Said nanotubes 23 or nanowires 26 are mono-dimensional structures, known in the art, schematically illustrated for example in figures 4-5.

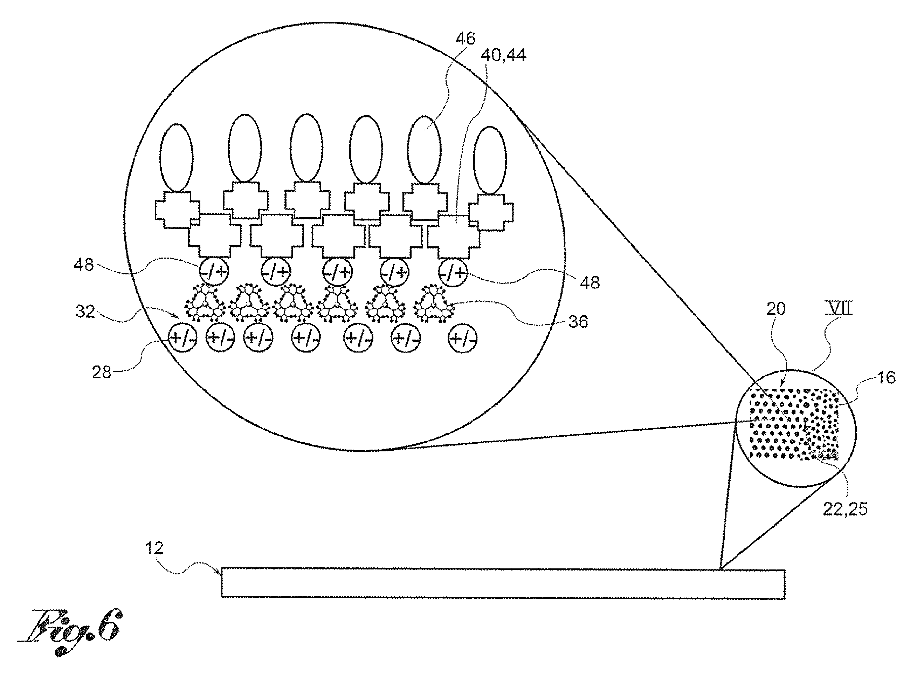

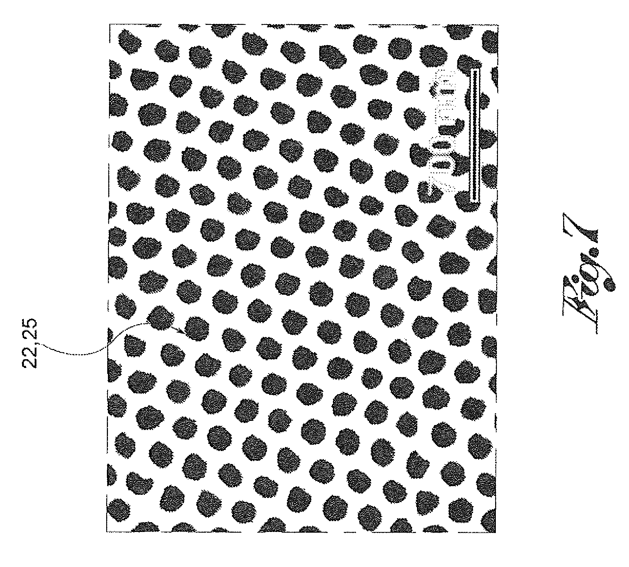

According to one embodiment said recesses 22 comprise nanoholes 25 (figures 6, 7). For example said nanoholes 25 may have a circular geometry.

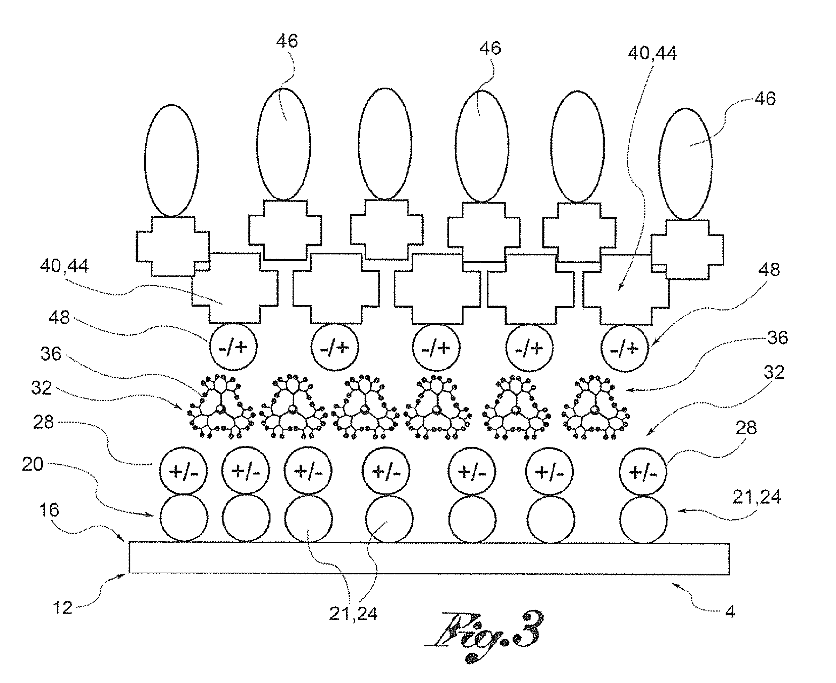

According to a further possible embodiment, on the side of the first covering 20, the protuberances 21 of the substrate 16 comprise nanometric spheres 24 (figure 3).

In particular the spheres have a high surface area: volume ratio, that is to say they have a large number of atoms on the surface which can create a plurality of chemical bonds.

According to one embodiment, such chemical bonds are of the ionic or covalent type.

Preferably, said nanometric spheres 24 are made from TiO2.

Preferably, the nanometric spheres 24 have a diameter of 100nm to 500nm.

A first covering 20 comprising first functional groups 28 suitable to form chemical bonds, is applied to said substrate 16.

Said nanometric spheres 24 are suitable for strengthening the bonds between the substrate 16 and the first functional groups 28.

According to one embodiment, such chemical bonds are of the ionic or covalent type.

According to one embodiment said substrate 16 is activated by the reaction of acids such as H3PO4, H2SO4 suitable for reacting with the material of the substrate so as to obtain the first functional groups 28 of the first covering 20 on it.

Preferably, the first functional groups 28 of the first covering 20 comprise OH-, COOH-, SH-, NH2- groups and the like.

On the same substrate 16 both protuberances 21,23,24,26 and recesses 22, 25 may be provided so as to improve the strength and quantity of bonds with said functional groups.

According to one embodiment a second covering 32 comprising nano-structured ramified polymers 36 is applied to the first covering 20, on the side opposite the substrate 16.

According to one embodiment, the second covering 32 comprises "dendrimer".

The ramified polymers 36 have the function of creating a plurality of chemical bonds with the first functional groups 28 of the first covering 20 as well as the function of creating bonds with further layers to superpose on the second covering 32.

Advantageously, an interface covering 40 comprising molecules 44 suitable for encouraging and /or discouraging the osseointegration of the prosthesis 4 depending on the greater or less osseointegration required of the interface wall 12 or portions thereof is applied to said second covering 32 on the side opposite the first covering 20.

In other words, the molecules 44 of the interface covering 40 are "calibrated" so as to provide specific biological stimuli when interfaced with bone cells 46; consequently depending on the type of molecules 44 selected and applied, the bone cells may be attracted or repelled.

Preferably, second functional groups 48 suitable to form chemical bonds are inserted between the interface covering 40 and the second covering 32.

According to one embodiment, such chemical bonds are of the ionic or covalent type.

For example, the second functional groups 48 comprise OH-, COOH-, SH-, NH2- groups and the like.

Said second functional groups 48 have the function of strengthening the bonds between the second covering 32 and the interface covering 40.

According to one embodiment the molecules 44 of the interface covering 40 comprise amino acids of the type RGD (Arg-Gly-Asp) and/or fibronectin suitable for favouring the osseointegration of the prosthesis 4.

According to a further embodiment, the molecules 44 of the interface covering 40 comprise monoclonal antibodies (TNF inhibitors) suitable for discouraging osseointegration.

The molecules 44 of the interface covering 40 may also comprise PEG and/or chitosan suitable for discouraging the risk of infections.

A method of making a prosthesis according to the present invention will now be described.

In particular the interface wall 12 having a substrate 16 is predisposed and the nanometric spheres are applied to the substrate 16.

For example, the substrate 16 is in titanium or titanium alloy and the nanometric spheres are in TiO2.

Such application of nanometric spheres is preferably performed by means of a PVD technique such as sputtering.

Then, according to one possible embodiment, the surface of the substrate is activated. For example, the surface of the substrate 16 is activated by applying acids, such as H3PO4, H2SO4. The first covering 20 comprising the first functional groups 28 suitable to form chemical bonds is obtained following such activation of the substrate 16. Said chemical bonds are for example of the ionic or covalent type.

The second covering 32 comprising nanostructures is then applied.

For example, said nanostructures comprise nanostructured ramified polymers 36. The interface covering 40 comprising molecules 44 is then applied to the second covering 2.

Second functional groups 48 suitable to form chemical bonds are preferably inserted between the interface covering 40 and the second covering 32.

Said chemical bonds are for example of the ionic or covalent type.

According to one embodiment different molecules 44 are applied to separate portions of the interface covering 40 depending on the desired level of osseointegration.

It is for example possible to encourage osseointegration in some portions of the prosthesis body 8 which need to integrate in the shortest time possible, and at the same time discourage such integration in different portions of the prosthesis body 8, for example so as to facilitate, or render less invasive, any subsequent removal or explant of the prosthesis.

Lastly, according to one possible embodiment, the interface covering 40 comprises PEG and/or chitosan molecules suitable to discourage the risk of infection following implant.

As may be appreciated from the description, the prosthesis according to the invention makes it possible to overcome the drawbacks presented in the prior art.

In particular the prosthesis according to the invention is of the multifunctional type, that is can be calibrated to behave differently towards the cells with which it comes into contact.

In fact, the interface covering may be modified by applying molecules able to encourage or discourage the osseointegration of the prosthesis or portions thereof. In fact, the type of molecules applied may attract or not the osteoblasts and cells which, after the implant, come into contact with the interface covering.

In other words, the molecules present on the interface covering are able to locally influence the behaviour of the prosthesis in relation to the cells with which it interfaces so as to accelerate osseointegration with specific portions of bone and/or discourage osseointegration with tissues with which it is preferable for the prosthesis not to bond; for example so as to facilitate any explants and substitutions of the prosthesis.

The particular succession of coverings on the substrate is able to contemporarily guarantee the multifunctionality of the prosthesis and also the solidness of the bonds and their resistance over time.

In other words, the molecules of the various coverings are bound to each other by numerous strong chemical bonds.

Moreover, by applying suitable molecules to the interface covering, it is possible to avoid side effects, such as infections or rejection phenomena of the prosthesis.

A person skilled in the art may make numerous modifications and variations to the prosthesis described above so as to satisfy contingent and specific requirements, all contained within the sphere of protection of the invention as defined by the appended claims.

## Claims

1. An interface wall (12) suitable for interfacing with at least one bone tissue,
- the interface wall (12) comprising a substrate (16) **characterised in that**
a first covering (20) comprising first functional groups (28) suitable to form chemical bonds, is applied to said substrate (16),
- wherein, on the side of the first covering (20) the substrate (16) comprises protuberances (21,23,24,26) and/or recesses (22,25) suitable to favour the attachment of said first functional groups (28),
- a second covering (32) comprising nanostructures being applied to said first covering (20), on the side opposite the substrate (16),
- an interface covering (40) comprising molecules (44) being applied to said second covering (32) on the side opposite the first covering (20),
- wherein second functional groups (48) suitable to form chemical bonds, so as to strengthen the bonds between the second covering (32) and the interface covering (40) are inserted between the interface covering (40) and the second covering (32).

2. The interface wall (12) according to claim 1, wherein said substrate (16) is made from titanium or titanium alloy.

3. The interface wall (12) according to claim 1 or 2 wherein said protuberances (21) comprise nanotubes (23) and/or nanowires (26).

4. The interface wall (12) according to claim 1, 2 or 3 wherein on the side of the first covering (20), said protuberances (21) comprise nanometric spheres (24) suitable to strengthen the bonds between the substrate (16) and the first functional groups (28).

5. The interface wall (12) according to claim 4, wherein said nanometric spheres (24) have a diameter of lOOnm to 500nm.

6. The interface wall (12) according to claim 4 or 5 wherein said nanometric spheres are made from Ti02.

7. The interface wall (12) according to any of the previous claims, wherein said recesses (22) comprise nanoholes (25).

8. The interface wall (12) according to any of the previous claims, wherein the first functional groups (48) of the first covering comprise OH-, COOH-, SH-, NH2- groups.

9. The interface wall (12) according to any of the previous claims, wherein the second covering (32) comprises nanostructured ramified polymers (36).

10. The interface wall (12) according to any of the previous claims, wherein said second covering (32) comprises "dendrimer".

11. The interface wall (12) according to any of the previous claims, wherein the second functional, groups (48) comprise OH-, COOH-, SH-, NH2- groups.

12. The interface wall (12) according to any of the previous claims, wherein the molecules (44) of the interface covering (40) comprise amino acids of the type RGD (Arg- Gly-Asp) and/or fibronectin suitable to favour the osseointegration.

13. The interface wall (12) according to any of the previous claims, wherein the molecules (44) of the interface covering (40) comprise monoclonal antibodies (TNF inhibitors) suitable to discourage osseointegration.

14. The interface wall (12) according to any of the previous claims, wherein the molecules (44) of the interface covering (40) comprise PEG and/or chifosan suitable to discourage the risk of infections.

15. Method of producing an interface wall (12) according to any of the previous claims, comprising the steps of:
- preparing an interface wall (12) having a substrate (16)
- applying protuberances (21,23,24,26) and/or recesses (22,25) to the substrate (16), suitable to favour the attachment of the first functional groups (28),
activating the surface of the substrate (16) by applying chemical substances, so as to obtain the first covering (20) comprising first functional groups (28) suitable to form chemical bonds,
applying the second covering (32) comprising nanostructures , inserting second functional groups (48) able to form chemical bonds between the interface covering (40) and the second covering (32), applying the interface covering (40) comprising molecules (44) to the second covering (32).
